**Europäisches Patentamt**

**European Patent Office**

**Office européen des brevets**

(19)

(11) Veröffentlichungsnummer : **0 293 029 B1**

(12) **EUROPÄISCHE PATENTSCHRIFT**

(45) Veröffentlichungstag der Patentschrift :
18.09.91 Patentblatt 91/38

(51) Int. Cl.⁵ : **C07D 475/04**

(21) Anmeldenummer : 88200864.2

(22) Anmeldetag : 22.04.88

(54) Verfahren zur Trennung von Folinsäure.

Verbunden mit 88903810.5/0314720
(europäische
Anmeldenummer/Veröffentlichungsnummer)
durch Entscheidung vom 27.08.90.
Die Akte enthält technische Angaben, die nach
dem Eingang der Anmeldung eingereicht
wurden und die nicht in dieser Patentschrift
enthalten sind.

(30) Priorität : 15.05.87 CH 1883/87

(43) Veröffentlichungstag der Anmeldung :
30.11.88 Patentblatt 88/48

(45) Bekanntmachung des Hinweises auf die
Patenterteilung :
18.09.91 Patentblatt 91/38

(84) Benannte Vertragsstaaten :
AT BE CH DE ES FR GB GR IT LI LU NL SE

(56) Entgegenhaltungen :
US-A- 2 688 018

(56) Entgegenhaltungen :
BIOCHEMISTRY, Band 20, 31. März 1981, Seiten 1837-1842, American Chemical Society, US; J. FEENY et al.: "Hydrogen-1 nuclear magnetic resonance study of the complexes of two diastereoisomers of folinic acid with dihydrofolate reductase"
JOURNAL OF THE AMERICAN CHEMICAL SOCIETY, Band 74, Nr. 16, 21. August 1952, Seiten 4215-4216, American Chemical Society, US; P.D. COSULICH et al.: "Diastereoisomers of leucovorin"

(73) Patentinhaber : EPROVA Aktiengesellschaft
Im Laternenacker 5
CH-8200 Schaffhausen (CH)

(72) Erfinder : Müller, Hans Rudolf
Beckenwäldli 18
CH-8207 Schaffhausen (CH)
Erfinder : Ulmann, Martin
Steigstrasse 339
CH-Dachsen (CH)
Erfinder : Conti, Josef
Winkelriesstrasse 22
CH-Schaffhausen (CH)
Erfinder : Mürdel, Günter
vor Hegin
W-7088 Tengen-Büsslingen (DE)

EP 0 293 029 B1

## Beschreibung

Die Erfindung betrifft ein Verfahren zur Herstellung von (6S)-Folinsäure bzw. von deren Salzen, insbesondere des Calcium-, Magnesium-, Kalium- und Natriumfolinats.

Folinsäure ist N-(5-Formyl-(6R,S)-5,6,7,8-tetrahydro-pteroyl)-L-glutaminsäure (5-CHO-(6R,S)-H$_4$PteGlu). N-(5-Formyl-(6S)-5,6,7,8-tetrahydropteroyl)-L-glutaminsäure (5-CHO-(6S)-H$_4$PteGlu) ist der Citrovorum-Faktor (= Wachstumsfaktor für Leuconostoc citrovorum).

Folinsäure enthält 2 asymmetrische Zentren. Dabei liegt aufgrund der Synthese der Folinsäure aus Folsäure, der N-(Pteroyl)-L-glutaminsäure, das im Glutaminsäure-Rest enthaltene optisch aktive C-Atom in der L-Form vor, während das durch Hydrierung der Doppelbindung in 5,6-Stellung des Pteroyl-restes entstandene optisch aktive C-Atom in Position 6 in der racemischen, der (6R,S)-Form, vorliegt. Synthetische Folinsäure (= Leucovorin) besteht demnach aus einer 1 : 1-Mischung von zwei Diastereomeren. Siehe dazu : J. Feeney et al, Biochemistry 20, 1837/38 (1981).

Leucovorin findet zunehmende Bedeutung als Arzneimittel zur Behandlung von megaloblastischer Folsäuremangel-Anämie, als Antidot zur Verstärkung der Verträglichkeit von Folsäure-Antagonisten speziell von Aminopterin und Methotrexat in der Krebstherapie (leucovorin rescue) und der Behandlung von Autoimmunkrankheiten wie Psoriasis und rheumatischer Arthritis sowie zur Verstärkung der Verträglichkeit von bestimmten Antiparasitika etwa Trimethoprim-Sulfamethoxazol in der Chemotherapie.

In natürlichen Vorkommen, z.B. in der Leber, findet man die Folinsäure nur in der S-Form. Die biochemische Wirkung von Leucovorin als Folsäure Cofaktor beruht auf dessen Gehalt an 5-CHO-(6S)-H$_4$PeGlu. Die inverse (R)-Form -5-CHO-(6R)-H$_4$PteGlu- dagegen wird kaum metabolisiert und langsam durch den Urin ausgeschieden. Sie ist biochemisch unwirksam. J.A. Straw et al, Cancer Research 44, 3114 (1984).

Es sind daher mehrfach Anstrengungen zur Trennung von 5-Formyl-(6R,S)-5,6,7,8-tetrahydropteroyl-L-glutaminsäure und zur asymmetrischen Synthese von 5-Formyl-(6S)-5,6,7,8-tetrahydropteroyl-Lglutaminsäure und Isolierung der physiologisch aktiven Form unternommen worden. D. Cosulich et al, J. Amer. chem. Soc. 74, 4215-16 (1952), US Patentschrift 2'688'018 haben versucht, die Trennung durch fraktionierte Kristallisation eines Erdalkali-salzes, z.B. des Calcium- oder Strontium-salzes, von 5-Formyl-(6R,S)-5,6,7,8-tetrahydropteroyl-L-glutaminsäure aus wässriger Lösung zu bewerkstelligen.

Unter den von B. Cosulich et al offenbarten Bedingungen lässt sich die gewünschte Trennung nicht realisieren. Bei der Kristallisation z.B. des Calcium-salzes von 5-Formyl-(6R,S)-5,6,7,8-tetrahydropteroyl-L-glutaminsäure aus Wasser bei pH 7-8 wird immer wieder reine 6R,S-Form erhalten, wie sich mittels chromatographischer Analyse an einer chiralen HPLC-Säule sowie anhand der optischen Drehung quantitativ nachweisen lässt. Dabei ist es unerheblich, ob man rohes oder reines Calcium-salz von 5-CHO-(6R,S)-H$_4$PteGlu zur Kristallisation einsetzt, stets wird die optisch reine (6R,S)-Form zurückerhalten. Eine Trennung und Anreicherung der (6S)-Form kann auch nicht erreicht werden, wenn man die übersättigte wässrige Lösung von Erdalkali-(6R,S)-Folinat mit authentischem Erdalkali-(6S)-Folinat impft. Damit blieb bis heute die asymmetrische Synthese als einzige Möglichkeit zur Gewinnung von N-(5-Formyl-(6S)-5,6,7,8-tetrahydropteroyl)-L-glutaminsäure. Die bisher bekannten Methoden der asymmetrischen Synthese von (6S)-Folinsäure sind jedoch zur Herstellung dieser Verbindung im technischen Massstab nicht geeignet. Es gibt daher bis heute keine technisch brauchbare Methode zur Gewinnung von (6S)-Folinsäure.

Es bestand somit die Aufgabe, eine einfache, technisch brauchbare und wirtschaftliche Methode zur Herstellung von (6S)-Folinsäure und ihrer Salze zu erarbeiten.

Es wurde nun überraschend gefunden, dass bei der Umkristallisation von Erdalkalisalzen von (6R,S)-Folinsäure, z.B. von Calcium-, Magnesium- oder Strontium-N-(5-Formyl-(6R,S)-5,6,7,8-tetrahydropteroyl)-L-glutaminat (= Erdalkali-(6R,S)-Folinat), vorzugsweise aus Wasser, in Gegenwart von anorganischen oder organischen Basen in alkalischem Milieu zunächst vorwiegend die (6S)Form auskristallisiert, wobei der Gehalt an der (6S)-Form im Kristallisat 85% und mehr erreichen kann.

Das mit der (6S)-Form stark angereicherte Erdalkali-Folinat mit einem hohen (6S)-Form-Gehalt lässt sich gegebenenfalls durch weitere Umkristallisation, vorzugsweise aus Wasser bei etwa neutralem pH, in optisch reines Erdalkali-(6S)-Folinat überführen.

Bei der Kristallisation kann die Ausbeute durch Zusatz von Erdalkaliionen, z.B. von Calcium-, Magnesium oder Strontiumchlorid, verbessert werden.

Gegenstand der Erfindung ist somit ein Verfahren zur Herstellung von (6S)-Folinsäure bzw. deren Salzen durch Umkristallisation von Erdalkalisalzen der (6R,S)-Folinsäure und gegebenenfalls Freisetzung der Säure aus den Erdalkalifolinaten und/oder gegebenenfalls Ueberführung in die Alkalisalze, das sich dadurch auszeichnet, dass die Umkristallisation in Gegenwart einer Base durchgeführt wird.

Gegenstand der Erfindung ist ferner ein Verfahren, bei dem das erhaltene Kristallisat in Gegenwart einer Base oder bei annähernd neutralem pH-Wert mindestens einer weiteren Umkristallisation unterworfen wird.

2

Nach einer bevorzugten Ausführungsform werden die Umkristallisationen in Gegenwart von zusätzlichen Erdalkali-Ionen durchgeführt. Als Ausgangsmaterial für das Verfahren sind sowohl reine Erdalkali-(6R,S)-Folinate als auch rohe Erdalkali-(6R,S)-Folinate geeignet.

Durch dieses Verfahren sind das Calcium- und Magnesium-salz von N-(5-Formyl-6S)-5,6,7,8-tetrahydropteroyl)-L-glutaminsäure erstmals technisch zugänglich geworden.

Als Erdalkalisalze von Folinsäure kommen in Betracht das Calcium-, Magnesium-, Strontium- und Barium-Folinat. Vorgezogen werden das Calcium- und Magnesium-Folinat, weil diese nach erfolgter Trennung direkt als Arzneimittel verwendbar sind, während etwa das Strontium- und vor allem das Barium-Salz anschliessend in ein anderes, physiologisch annehmbares, Salz umgewandelt werden.

Als anorganische oder organische Basen kommen beispielsweise in Betracht :

Alkalihydroxide wie Natrium-, Kalium- und Lithium-hydroxid,

Erdalkalihydroxide wie insbesondere Calcium- und Magnesium-hydroxid, Ammoniak, Hydrazin, wasserlösliche organische Basen,

insbesonders einfache primäre, sekundäre, tertiäre Amine, z.B. Methylamin, Dimethylamin, Trimethylamin, Ethylamin, Diethylamin, Triethylamin, Propylamin, Dipropylamin, Methylethylamin,

Aminoalkohole, z.B. Ethanolamin, Diethanolamin, Triethanolamin, Propanolamine, Butanolamine, Dihydroxypropylamine (2,3-Dihyroxypropylamin, Serinol), Trihydroxybutylamine (Tris-(hydroxymethyl)-aminomethan), Glucamin, N-Methyl-glukamin,

heterocyklische Amine, z.B. Pyrrolidin, Piperidin, Morpholin oder Piperazin.

Die erste Umkristallisation nach dem erfindungsgemässen Verfahren wird bei einem pH-Wert zwischen 8,5 und 12, vorzugsweise zwischen 9,0 und 10,5, durchgeführt. Bei tieferem pH erfolgt keine deutliche Anreicherung der (6S)-Form im Kristallisat, bei höherem pH wird die Stabilität der Folinsäure merklich geringer und zudem sind die Erdalkalisalze kaum noch zur Kristallisation zu bringen.

Zur Entfernung restlicher Mengen von (6R)-Folinat aus Erdalkali-(6S)-Folinat können weitere Umkristallisationen auch in annähernd neutralem Milieu z.B., im pH-Bereich von 6,5 bis 8,5, durchgeführt werden. Bei den Umkristallisationen kann durch Zusatz von entsprechenden Erdalkali-Ionen das Löslichkeitsprodukt rascher erreicht und damit die Ausbeute gesteigert werden. Die Erdalkali-Ionen, vorzugsweise Ca, Mg, Sr, werden in Form beliebiger leicht löslicher Salze zugefügt, beispielsweise in Form der Chloride, Sulfate oder Nitrate. Sie werden in der Regel in der 0,2 bis 4fachen Menge des Folinats eingesetzt.

Gegenstand der Erfindung sind auch die durch das erfindungsgemässe Verfahren erstmals technisch zugänglichen (6S)-Folinate, insbesondere das Calcium-folinat und die (6S)-Folinsäure sowie, unabhängig von der Methode seiner Herstellung, das neue Magnesiumsalz und das aus den nun gut zugänglichen Erdalkali-(6S)-Folinaten, z.B. durch Umsalzen leicht erhältliche neue Natrium- und Kalium-(6S)-Folinat.

Das Magnesium-(6S)-Folinat ist von herausragender Bedeutung, weil es aufgrund seiner vergleichsweise guten Wasserlöslichkeit von mehr als 2 g/100 ml und seiner hohen Verträglichkeit das geeignete Ausgangsmaterial für die Herstellung von Injektionslösungen bildet. Noch mehr gilt dasselbe für das Natrium- und Kalium-(6S)-Folinat.

Das Calcium-(6S)-Folinat vermag bei 20°C nur 0.95%ige wässrige Lösungen zu bilden, was die Herstellung von Injektionslösungen erschwert.

Beispiele zur Illustrierung der Erfindung :

Beispiel 1

Calcium-(6S)-Folinat

1. Kristallisation :

100 g rohes Calcium-(6R,S)-Folinat in ca 1 Liter warmem Wasser von 50-60°C werden mit 12-36 g Cal-ciumchlorid (CaCl$_2$ · 2H$_2$O) versetzt, bei 30°C durch Zusatz von wässrigem Ammoniak (25%ig) auf pH 10 eingestellt und bei 18°C kristallisieren gelassen. Nach 18-20 Stunden wird das ausgeschiedene Produkt abfiltriert, mit verdünnter Calciumchlorid-Lösung und danach mit wasserfeuchtem Ethanol gewaschen. Man erhält 41 g Calcium-Folinat enthaltend 88% Calcium-(6S)-Folinat und 12% Calcium-(6R)-Folinat. Optische Ausbeute 72%.

2. Kristallisation :

40 g rohes Calcium-(6R,S)-Folinat aus der 1. Kristallisation enthaltend 88% (6S)-Folinat, werden bei 55-60°C in Wasser gelöst und langsam mit wässriger Salzsäure (20%ig) bis zum pH 6,1 und mit 40 bis 160 g Calciumchlorid versetzt. Bei 55°C wird durch Zusatz von Natronlauge das pH der Lösung auf 7 bis 7.5. eingestellt. Bei etwa 35°C wird mit authentischem Calcium-(6S)-Folinat geimpft und bei 18-20°C das Produkt kristallisieren gelassen. Nach etwa 40 Stunden wird das auskristallisierte Produkt abfiltriert, mit wässrigem Ethanol gewaschen und getrocknet. Man erhält 30,4 g Calcium-(S)-Folinat mit einem Gehalt an (S)-Folinat von 98%. Optische Ausbeute : 79-81%.

3. Kristallisation :

10 g Calcium-(6S)-Folinat mit einem Gehalt an (6S)-Form von 94-98% werden in heissem Wasser gelöst, mit 10 g Calciumchlorid versetzt und bei pH 7.0 bis 7.5 und 18-20°C kristallisieren gelassen. Nach 3 bis 4 Tagen wird das Produkt abfiltriert, mit wenig Wasser und mit feuchtem Ethanol gewaschen und getrocknet. Man erhält 8 g reines Calcium-(6S)-Folinat.

```
Gehalt an     Calcium-(6S)-Folinat  =  99 - 100 Flächen%


Löslichkeit in Wasser:          0,95 g/100 ml bei 20°C und

                                1.5  g/100 ml bei 40°C

Spezifische Drehung [α]²⁰_D  = - 15° (bezogen auf wasserfreies
                                      Ca-Salz)


Anmerkungen:
Gehalt an CaFolinat:            Bestimmt mittels HPLC gegen Standard.
Gehalt an (6S)-Form:            Bestimmt mittels HPLC unter Verwendung
                                einer chiralen Säule
                                (Resolvosil-BSA-7).
```

Beispiel 2

Trennung von Calcium-(6R,S)-Folinat durch Kristallisation in Gegenwart von verschiedenen Basen

1. Umkristallisation in Gegenwart von Basen.

30 g Calcium-(6R, S)-Folinat werden bei 50°C in 200-300 ml Wasser gelöst, bei 30-40°C mit 0,5-0,6 Aequivalent Base pro Mol Calcium-(6R, S)-Folinat versetzt.
Die Lösung wird bei Raumtemperatur während 5 bis 17 Stunden gerührt. Gewöhnlich tritt bald spontane Kristallisation ein.
Das Kristallisat wird abfiltriert, mit wenig 5%iger Calciumchlorid-Lösung und mit Ethanol gewaschen und getrocknet.
Die Ergebnisse sind aus der Tabelle 1 ersichtlich.

Tabelle 1

| Base | pH | Gehalt an (6S)-Form Flächen% | Ca-Folinat·5H$_2$O Gew.% | optische Ausbeute % |
|------|-----|------|------|------|
| Kein Basenzusatz | 7.5 | 50.1 | 99 | keine Auftrennung |
| Natriumhydroxid | 8.5 | 52 | 101 | beginn. Auftrenng |
| NaOH | 10 | 80 | 96 | 70 |
| Magnesiumhydroxid | 10 | 72 | 97.2 | 60 |
| Calciumhydroxid | 10.2 | 79 | 93.4 | 65 |
| Aminobutanol | 9.6 | 71 | 96.5 | 70 |
| Ethanolamin | 10 | 85 | 97.5 | 75 |
| Diethanolamin | 9.7 | 75 | 97.2 | 60 |
| Serinol | 9.6 | 75 | 98.2 | 75 |
| Methylamin | 10 | 79 | 96.5 | 76 |
| Ethylamin | 9.9 | 82 | 97.5 | 78 |
| Ammoniak | 10 | 84 | 98.9 | 72 |
| Hydrazin | 10 | 83 | 97 | 72 |
| Kaliumhydroxid | 9.5 | 77 | 98.2 | 70 |

2. Umkristallisation der nach 1. erhaltenen rohen Calcium-(6S)-Folinate.

Die nach vorstehender Methode 1 erhaltenen rohen Calcium-(6S)-Folinate werden unter Zusatz von 1-4 Teilen Calciumchlorid bei pH 6.5 bis 7.5 aus Wasser umkristallisiert. Eine etwas schwerer lösliche Fraktion wird dabei durch Filtration abgetrennt. Aus dem Filtrat kristallisiert nach Einengen und Abkühlen reines Calcium-(6S)-Folinat.
Gehalt an Calcium-(6S)-Folinat = 99.9 Flächen%

Beispiel 3

Magnesium-(6S)-Folinat

1. Kristallisation :

30 g Magnesium-(6R,S)-Folinat, hergestellt aus einer wässrigen Lösung von Natrium-(6R,S)-Folinat durch Fällung mit Magnesiumchlorid, werden in heissem Wasser gelöst, mit 100 g Magnesiumchlorid versetzt und durch Zusatz von wässrigem Natriumhydroxid auf pH 10 gestellt. Nun wird unter Rühren auf 16-18°C abgekühlt. Nach einigen Tagen wird das auskristallisierte rohe Magnesium-(6S)-Folinat abfiltriert, mit Ethanol gewaschen und getrocknet. Man erhält ein Magnesium-Folinat mit einem Gehalt von 80% an (S)-Folinat.

2. Umkristallisation :

Durch Umkristallisation bei annähernd neutralem pH aus wenig Wasser unter Zusatz von Magnesiumchlorid erhält man aus dem rohen reines Magnesium-(6S)-Folinat mit einem Gehalt an (6S)-Folinat von mehr als 95% der Theorie.
Löslichkeit in Wasser : 2,4 g/100 ml bei 20°C.

5

## Beispiel 4

### Natrium-(6S)-Folinat

Eine praktisch gesättigte wässrige Lösung von Calcium-(6S)-Folinat wird durch eine Ionenaustauscher-Säule, die mit Kationenaustauscherharz in der Na(+)-Form, z.B. mit Amberlite IR-120, Na(+)-Form, beschickt ist, perkolieren gelassen. Das Eluat wird konzentriert. Durch Zusatz von Ethanol wird das Natrium-(6S)-Folinat ausgefällt.
Natrium-(6S)-Folinat ist leicht löslich in Wasser.
Alternativ lässt sich das Natrium-(6S)-Folinat auch durch Lösen von (6S)-Folinsäure in der äquivalenten Menge von Natriumhydroxid gewinnen.
Die dazu erforderliche (6S)-Folinsäure wird nach Beispiel 6 erhalten.

## Beispiel 5

### Kalium-(6S)-Folinat

Diese Verbindung wird erhalten, indem man nach Beispiel 6 erhaltene (6S)-Folinsäure in der äquivalenten Menge von wässrigem Kaliumhydroxid auflöst.
Aus seinen konzentrierten wässrigen Lösungen lässt sich durch Versetzen mit Ethanol, Isopropanol oder Aceton das Kalium-(6S)-Folinat ausfällen.
Kalium-(6S)-Folinat ist leicht löslich in Wasser.

## Beispiel 6

### (6S)-Folinsäure

Eine wässrige Lösung von Calcium-(6S)-Folinat wird vorsichtig mit verdünnter Salzsäure versetzt, wobei die (6S)-Folinsäure ausfällt und durch Filtration gewonnen wird.
(6S)-Folinsäure ist kaum löslich in Wasser.

## Patentansprüche

1. Verfahren zur Herstellung von (6S)-Folinsäure bzw. deren Salzen durch Kristallisation von Erdalkalisalzen der (6R,S)-Folinsäure und gegebenenfalls Freisetzung der Säure aus den Erdalkalifolinaten und/oder gegebenenfalls Uberführung in die Alkalisalze, dadurch gekennzeichnet, dass die Kristallisation in Gegenwart einer Base durchgeführt wird.
2. Verfahren nach Anspruch 1, dadurch gekennzeichnet, dass das erhaltene Kristallisat in Gegenwart einer Base oder bei annähernd neutralem pH-Wert mindestens einer weiteren Kristallisation unterworfen wird.
3. Verfahren nach Anspruch 1 und 2, dadurch gekennzeichnet, dass man die Kristallisationen in Gegenwart von zusätzlichen Erdalkali-Ionen durchführt.
4. Magnesium-Salz der (6S)-Folinsäure.
5. Natrium- und Kalium-Salz der (6S)-Folinsäure.

## Claims

1. Process for the preparation of (6S)-folic acid or its salts by crystallisation of alkaline earth metal salts of (6R,S)-folic acid and, if desired, release of the acid from the alkaline earth metal folates and/or, if desired, conversion into the alkali metal salts, characterised in that the crystallisation is carried out in the presence of a base.
2. Process according to Claim 1, characterised in that the crystallisate obtained is subjected to at least one further crystallisation in the presence of a base or at approximately neutral pH.
3. Process according to Claims 1 and 2, characterised in that the crystallisation is carried out in the presence of additional alkaline earth metal ions.
4. Magnesium salt of (6S)-folic acid.
5. Sodium salt and potassium salt of (6S)-folic acid.

6

**Revendications**

1. Procédé pour préparer l'acide folinique (6S),ou ses sels, par cristallisation de sels alcalino-terreux de l'acide folinique (6R,S), et, le cas échéant, la libération de l'acide à partir des folinates alcalino-terreux, et/ou éventuellement la transformation en sels alcalins, ce procédé étant caractérisé en ce que la cristallisation est effectuée en présence d'une base.

2. Procédé selon la revendication 1, caractérisé en ce que le produit cristallisé obtenu est soumis à au moins une autre cristallisation, en présence d'une base ou à un pH approximativement neutre.

3. Procédé selon les revendications 1 et 2, caractérisé en ce que la cristallisation est effectuée en présence d'ions alcalino-terreux additionnels.

4. Sel de magnésium de l'acide folinique (6S).

5. Sels de sodium et de potassium de l'acide folinique (6S).